(19) Europäisches Patentamt

European Patent Office

Office européen des brevets

(11) **EP 1 417 492 B1**

(12) **EUROPÄISCHE PATENTSCHRIFT**

(45) Veröffentlichungstag und Bekanntmachung des Hinweises auf die Patenterteilung:
**28.06.2006 Patentblatt 2006/26**

(21) Anmeldenummer: **02794751.4**

(22) Anmeldetag: **05.08.2002**

(51) Int Cl.:
*G01N 33/543* (2006.01)    *G01N 27/327* (2006.01)
*G03F 7/34* (2006.01)    *B01J 19/00* (2006.01)

(86) Internationale Anmeldenummer:
**PCT/EP2002/008724**

(87) Internationale Veröffentlichungsnummer:
**WO 2003/016912 (27.02.2003 Gazette 2003/09)**

(54) **"LAB ON CHIP" AUS PHOTORESISTMATERIAL FÜR MEDIZINISCH DIAGNOSTISCHE ANWENDUNG**

"LAB ON CHIP" FROM PHOTORESIST MATERIAL FOR MEDICAL DIAGNOSTIC APPLICATIONS

LABORATOIRE SUR PUCE EN RESINE PHOTOSENSIBLE POUR DES APPLICATIONS DE DIAGNOSTIC MEDICAL

(84) Benannte Vertragsstaaten:
**AT BE BG CH CY CZ DE DK EE ES FI FR GB GR IE IT LI LU MC NL PT SE SK TR**

(30) Priorität: **13.08.2001 DE 10139742**

(43) Veröffentlichungstag der Anmeldung:
**12.05.2004 Patentblatt 2004/20**

(73) Patentinhaber:
• **Universität Freiburg**
  **79100 Freiburg (DE)**
• **Moser, Isabella**
  **79356 Eichstetten (DE)**

(72) Erfinder:
• **MOSER, Isabella**
  **79356 Eichstetten (DE)**
• **JOBST, Gerhard**
  **79356 Eichstetten (DE)**
• **GAMP, Thomas**
  **72458 Albstadt (DE)**

(74) Vertreter: **Müller-Boré & Partner**
**Patentanwälte**
**Grafinger Strasse 2**
**81671 München (DE)**

(56) Entgegenhaltungen:
**WO-A-00/32678**        **WO-A-00/33084**
**US-A- 5 212 050**

• JOBST G ET AL: "Rapid liver enzyme assay with miniaturized liquid handling system comprising thin film biosensor array" SENSORS AND ACTUATORS B, ELSEVIER SEQUOIA S.A., LAUSANNE, CH, Bd. 44, Nr. 1-3, 1. Oktober 1997 (1997-10-01), Seiten 377-380, XP004117166 ISSN: 0925-4005
• JOBST G ET AL: "Application Of Miniaturized Liquid Handling System With Integrated Biosensor Array For Milk Analysis" INT CONF SOLID STATE SENSORS ACTUATORS EUROSENSORS IX PROC 1995; , Bd. 2, 25. Juni 1995 (1995-06-25), Seiten 473-474, XP010305057

**Beschreibung**

Die vorliegende Erfindung betrifft eine Vorrichtung zur Durchführung von Immunoassays ("BioChip" bzw. "Lab on Chip"), ein Verfahren zu deren Herstellung sowie die Verwendung der Vorrichtung zur Durchführung von Immunoassays, beispielsweise EIA und ELISA. Ferner betrifft die vorliegende Erfindung die Verwendung von durch Photolithographie strukturierbaren Trockenfotoresistfilmen auf Basis eines Materials mit funktionalen chemischen Gruppen zur Immobilisierung von Biomolekülen.

Für die Biowissenschaften und die medizinische Diagnostik ist die Detektion (bio)chemischer Reaktionen, d.h. die Detektion biologisch relevanter Moleküle in definiertem Untersuchungsmaterial von herausragender Bedeutung. In diesem Rahmen wird die Entwicklung von sogenannten BioChips stetig vorangetrieben. Bei BioChips handelt es sich üblicherweise um miniaturisierte hybride Funktionselemente mit biologischen und technischen Komponenten, insbesondere auf einer Oberfläche (Außenoberfläche und/oder Innenoberfläche) immobilisierte Biomoleküle, die als spezifische Interaktionspartner dienen. Häufig weist die Struktur dieser Funktionselemente Reihen und Spalten auf. Man spricht dann von sogenannten "Chip-Arrays". Da tausende von biologischen bzw. biochemischen Funktionselementen auf einem Chip angeordnet sein können, müssen diese in der Regel mit mikrotechnischen Methoden angefertigt werden. Als biologische und biochemische Funktionselemente kommen insbesondere DNA, RNA, PNA, (bei Nukleinsäuren und ihren chemischen Derivaten können z.B. Einzelstränge, Triplex-Strukturen oder Kombinationen hiervon vorliegen), Saccharide, Peptide, Proteine (z.B. Antikörper, Antigene, Rezeptoren), Derivate der kombinatorischen Chemie (z.B. organische Moleküle), Zellbestandteile (z.B. Organellen), Zellen, Mehrzeller sowie Zellverbände in Frage.

Im allgemeinen haben BioChips eine 2D-Basisfläche für das Beschichten mit biologisch oder biochemisch funktionellen Materialien. Die Basisflächen können beispielsweise auch von Wänden einer oder mehrerer Kapillaren bzw. Kanälen gebildet sein. Eine Weiterführung der Geometrie ist eine 3D-Struktur, bei der die Analyse und gegebenenfalls auch Manipulation respektive Steuerung von Reaktionen in einer 2D-Anordnung erfolgen. Die derzeit im Stand der Technik verfügbaren Verfahren zur Herstellung von BioChips erfordern dabei üblicherweise vor dem ortsspezifischen Binden bzw. Verknüpfen von biologischen bzw. biochemischen Funktionselementen eine chemische Funktionalisierung der Mikrostrukturoberflächen. Dies kann insbesondere durch das Aktivieren photosensitiver Gruppen auf vorbestimmten Bereichen einer Substratoberfläche mittels ortsspezifischer Belichtung des Substrats mit einer Belichtungsmatrix erreicht werden (siehe beispielsweise DE 199 40 752 A1). Die zur Ankopplung von Biomolekülen erforderliche chemische Funktionalisierung der Mikrostrukturoberflächen erschwert jedoch die produktionstechnischen Anforderungen an die Herstellung derartiger BioChips erheblich, was derartige Verfahren nicht nur weniger zuverlässig, sondern auch äußerst kostenaufwendig macht. Werden die Biomoleküle mittels Adsorptionstechniken aufgebracht, stellt auch dies einen hohen Aufwand an die Oberflächenbeschichtung dar.

Der vorliegenden Erfindung liegt somit die Aufgabe zugrunde, ein einfaches, flexibles und kostengünstiges Verfahren zur Herstellung von sogenannten "Lab on Chips" bzw. "BioChips" bereitzustellen, das insbesondere das ortsspezifische Verknüpfen von biologischen bzw. biochemischen Funktionselementen erleichtern soll, d.h: die Probleme, die mit der vor dem ortsspezifischen Verknüpfen von biologischen bzw. biochemischen Funktionselementen erforderlichen chemischen Funktionalisierung der Mikrostrukturoberflächen verbunden sind, vermeiden soll.

Diese Aufgabe wird durch die in den Ansprüchen gekennzeichneten Ausführungsformen gelöst.

Insbesondere wird ein Verfahren zur Herstellung einer Vorrichtung zur Durchführung von Immunoassays bereitgestellt, umfassend:

(a) Bereitstellen eines mit vorbestimmten Vertiefungen und/oder Durchgangslöchern mikrostrukturierten Substrats,
(b) Aufbringen von mindestens einem Film eines Trockenfotoresistmaterials mit funktionalen chemischen Gruppen auf das Substrat,
(c) Belichten des Trockenfotoresistfilms unter Verwendung einer Fotoschablone mit einem vorbestimmten Muster,
(d) Entwickeln des Trockenfotoresistfilms,
(e) Wiederholen des Schritts (b), des Schritts (c) unter Verwendung einer Fotoschablone mit einem anderen vorbestimmten Muster und des Schritts (d), so dass Substrat und Trockenfotoresistmaterial miteinander eine Kapillarstruktur bzw. Kapillarkanalstruktur mit mindestens einem Einlass und Auslass bilden,
(f) ortsspezifisches Anbinden von Biomolekülen an mindestens einen Bereich der von dem Trockenfotoresistmaterial gebildeten Innenoberflächen der in Schritt (e) erzeugten Kanalstruktur durch chemische Bindung über die funktionalen Gruppen des Trockenfotoresistmaterials, wodurch eine Vorrichtung zur Durchführung von Immunoassays erhalten wird.

Die vorliegende Erfindung stellt eine neue Technologieplattform zur kostengünstigen, flexiblen und zuverlässigen Herstellung von Vorrichtungen, die sich insbesondere zur Durchführung von Immunoassays (sogenannte "Lab on Chips" bzw. "BioChips") eignen, dar.

Die Figuren zeigen:

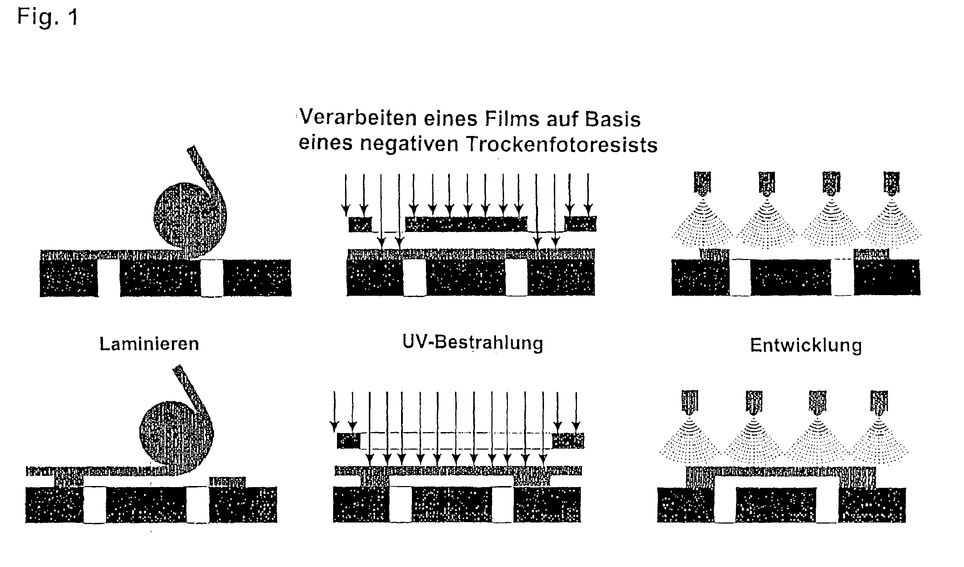

Fig. 1 zeigt ein beispielhaftes Schema zur Herstellung eines Kapillarkanalsystems im Rahmen des erfindungsgemäßen Verfahrens.

Fig. 2 zeigt ein beispielhaftes Schema zur Durchführung eines ELISA unter Verwendung eines erfindungsgemäß hergestellten Kapillarkanalsystems.

Die in dem erfindungsgemäßen Verfahren verwendeten Trockenfotoresistfilme bzw. -folien basieren auf fotostrukturierbaren Polymeren, die funktionale chemische Gruppen aufweisen, die befähigt sind, mit entsprechenden Biomolekülen eine chemische Bindung einzugehen. Vorzugsweise wird im Rahmen des erfindungsgemäßen Verfahrens ein Trockenfotoresistfilm auf Basis eines negativen Fotoresists eingesetzt. Insbesondere werden Trockenfotoresistfilme auf Basis von Polymermaterialien eingesetzt, die chemische funktionale Gruppen enthalten, ausgewählt aus Carbonsäuren, Carbonsäureanhydriden, Carbonsäurechloriden, Aldehyden, Glyoxalen, N-Hydroxysuccinimidester, Hydraziden, Imidaten, Isothiocyanaten, Isocyanaten, Maleinimiden, Halogenchinonen, Epoxiden, Aziridinen, Acylaziden, Phenolen, Aminogruppen, Thiolgruppen, Hydroxylgruppen, Sulfhydryl-reaktivem Brom und Iod sowie Biotingruppen. Vorzugsweise sind die chemischen funktionalen Gruppen Thiolgruppen (-SH) und davon abgeleitete Gruppen wie z.B. Pyridylthiogruppen, Carbonsäuregruppen (-COOH) und davon abgeleitete Gruppen wie beispielsweise Carbonsäureanhydridgruppen wie z.B. Maleinsäureanhydrid oder Bernsteinsäureanhydrid, Imidestergruppen wie z.B. N-Hydroxysuccinimidestergruppen, und Carboxylatgruppen. Besonders bevorzugt sind Carbonsäuregruppen. In einer bevorzugten Ausführungsform der vorliegenden Erfindung ist der Trockenfotoresistfilm auf Basis eines Gemisches von Polymeren und gegebenenfalls Oligomeren und/oder Monomeren, wobei mindestens eines der Polymere, Oligomere oder Monomere Thiol-, Carbonsäure-, Anhydrid-, Säure-amid- oder Imidestereinheiten, vorzugsweise Acrylsäure-, Methacrylsäure-, Ma-leinsäureanhydrid-, Maleinsäureimid- oder N-Hydroxysuccinimidestereinheiten aufweist. Die in einem derartigen Gemisch verwendeten Polymere können Blockpolymere, Copolymere oder Pfropfcopolymere, insbesondere Copolymere, vorzugsweise statistische oder Blockcopolymere sein, die vorzugsweise Carbonsäure-, Anhydrid-, Säureamid- oder Imidestereinheiten enthalten. Beispielsweise können hier Fotoresistmaterialien auf Styrol/Maleinsäureanhydrid-Copolymerbasis angeführt werden. Selbstverständlich kann ein derartiges Gemisch weitere Hilfsstoffe, wie z.B. Füller, Vernetzer, Weichmacher und Photoinitiatoren umfassen, wie es einem Fachmann auf dem Gebiet der Trockenfotoresistfilme wohlbekannt ist. Im Rahmen der vorliegenden Erfindung ist auch das Vermischen bzw. Compoundieren eines wie vorstehend ausgeführten Trockenfotoresistmaterials auf Polymerbasis mit einem Monomer oder Oligomer, welches einerseits befähigt ist, in der durch Belichten induzierten Vernetzungsreaktion in das Polymernetzwerk eingebaut zu werden, und andererseits weiter eine der vorstehend angeführten funktionalen chemischen Gruppen aufweist, die befähigt sind, mit entsprechenden Biomolekülen eine chemische Bindung einzugehen, eingeschlossen. Beispielhaft können hier γ-Maleimidobutansäure-N-hydroxysuccinimid, γ-Maleimidocapronsäure-N-hydroxysuccinimid oder N-Acryloxysuccinimid genannt werden.

Als im Rahmen der vorliegenden Erfindung verwendbare Trockenfotoresistfilmmaterialien können hier beispielsweise Vacrel® oder Riston® , jeweils vertrieben von Dupont, angeführt werden.

Da bereits das Fotoresistmaterial, welches in Kombination mit dem Substrat zur Herstellung der Kapillarstrukturen mittels fotolithographischer Verfahren verwendet wird, funktionale chemische Gruppen enthält, liegt an der Oberfläche der das Kapillarkanalsystem bildenden Trockenfotoresistschichten, wie auch im Material selbst, eine hohe Konzentration an funktionalen chemischen Gruppen, insbesondere freien Carbonsäuregruppen vor, was eine dauerhafte, kovalente chemische Ankopplung von Biomolekülen an diese Oberfläche ermöglicht. Das Fotoresistmaterial kann dabei in besonders vorteilhafter Weise zur Herstellung entsprechender Mikrostrukturen, d.h. Kanälen bzw. Kapillaren, nach Techniken verarbeitet werden, wie sie insbesondere in der Leiterplattenherstellungstechnik eingesetzt werden.

Das mit vorbestimmten Vertiefungen und/oder Durchgangslöchern mikrostrukturierte Substrat bzw. Träger kann beispielsweise ein Leiter- bzw. Halbleitersubstrat, ein Glassubstrat oder ein Kunststoffsubstrat oder aber auch ein unstrukturiertes bzw. bereits strukturiertes Trockenfotoresistmaterial sein. Die vorbestimmten Vertiefungen und/oder Durchgangslöchern können dabei beispielsweise mittels herkömmlicher photolithographischer Techniken und Ätzprozesse erzeugt werden. Im Falle eines bereits strukturierten Trockenfotoresistmaterials kann dieses durch vorangegangene Strukturierung eines Trockenfotoresistfilms erzeugt werden.

Durch wiederholtes Laminieren und Photostrukturieren unter Verwendung unterschiedlicher Fotomasken können durch das erfindungsgemäße Verfahren geschlossene Kapillarstrukturen bzw. mindestens Teile von Kanälen bzw. Kapillaren, die mindestens einen Einlass und Auslass aufweisen, hergestellt werden. Typische Dimensionen derartiger in Schritt (e) des erfindungsgemäßen Verfahrens erzeugter Kanalstrukturen sind beispielsweise Höhen im Bereich von 10 bis 100 μm, Breiten im Bereich von 10 bis 500 μm und Längen im Bereich von 10 bis 1000 mm. Die Kanäle können dabei gerade oder mäanderförmig sowie durchgehend oder kammartig geformt sein. Darüberhinaus können sie einzeln oder in ein- oder zweidimensionalen Arrays angeordnet sein. Ferner können sie in einer Ebene oder in mehreren Ebenen übereinander angeordnet sein. Mit dem erfindungsgemäßen Verfahren sind daher auch dreidimensionale Vorrichtungen (Arrays) erhältlich. Wenn die Kapillarstruktur(en) in mehreren Ebenen übereinander angeordnet sind, können die Kanäle über vertikale Kanäle miteinander verbunden sein. Nulldimensionale (d.h. eine einzelne Kapillare), eindimensionale oder zweidimensionale Arrays können vertikal gestapelt werden bzw. in einer derartigen Konfiguration auch über vertikale Öffnungen fluidisch miteinander verbunden sein.

In einer Ausführungsform des erfindungsgemäßen Verfahrens können vor dem Schritt (f) die funktionalen chemischen Gruppen aktiviert werden. Wenn die Trockenfotoresistfilme auf Basis von Polymermaterialien sind, die Carbonsäuregruppen enthalten, kann die Aktivierung beispielsweise durch Umsetzung mit einer Carbodiimid-Verbindung, wie Dicyclohexylcarbodiimid (DCC), durchgeführt werden. Ein solche Carbodiimid-Lösung kann durch mindestens Teile des Kanalsystems gepumpt werden, wodurch die Carbonsäuregruppen aktiviert werden. Anschließend werden die derart aktivierten Innenoberflächen an den Stellen des Kanalsystems, die durch das Trockenfotoresistmaterial gebildet werden, durch chemische Bindung mit entsprechend gewählten Biomolekülen, wie z.B. einem Antikörper, mit diesen beschichtet.

In einer Ausführungsform der vorliegenden Erfindung kann der Schritt (f) mittels Tauch- oder Gießverfahren durchgeführt und anschließend die Kanäle der Kapillarstruktur mit einer oberen Lage von mindestens einem Trockenfotoresistfilm verschlossen werden. Alternativ kann im Zuge des Schritts (e) mit Ausnahme der als Einlässe und Auslässe vorgesehenen Kanäle die Kapillarstruktur verschlossen und anschließend der Schritt (f) mittels Pumptechniken durchgeführt werden. Des weiteren kann in Schritt (f) auch eine Lösung von Biomolekülen unter Ausnutzen der Kapillarkraft mittels mindestens einer Nadel oder Nadelanordnung in eine oder mehrere Kapillare der in Schritt (e) erhaltenen Kapillarstruktur beschickt bzw. gefüllt werden. Beispielsweise kann ein Überschuss an Flüssigkeit mit einer Nadel oder einem Instrument, welches eine Multiplizität solcher Nadeln und Flüssigkeiten aufweist, über eine vertikale Öffnung einer solchen Kapillare so beschickt werden, dass sich diese Kapillare durch die Kapillarwirkung mit Flüssigkeit füllt. Nach der gewünschten Reaktionszeit kann die Flüssigkeit durch Anlegen von Überdruck an eine Öffnung der Kapillare entfernt werden. Vorteilhaft ist auch eine Ausführungsform, in welcher die Kapillare Öffnungen aufweist, die nur für die Beschichtung bzw. Verknüpfung mit Biomolekülen Verwendung finden und welche nach der Beschichtung verschlossen werden, beispielsweise mit Trockenresistfilm oder einer selbstklebenden Folie bzw. Klebeband.

Als Biomoleküle, die in Schritt (f) ortsspezifisch und selektiv durch direkte chemische Bindung über die funktionalen Gruppen des Trockenfotoresistmaterials an mindestens einen Bereich der von dem Trockenfotoresistmaterial gebildeten Innenoberflächen der in Schritt (e) erzeugten Kapillarstruktur gekoppelt bzw. gebunden werden, kommen insbesondere DNA, RNA, PNA, (bei Nukleinsäuren und ihren chemischen Derivaten können z.B. Einzelstränge, Triplex-Strukturen oder Kombinationen hiervon vorliegen), Saccharide, Peptide, Proteine (z.B. Antikörper, Antigene, Rezeptoren), Derivate der kombinatorischen Chemie (z.B. organische Moleküle), Zellbestandteile (z.B. Organellen), Zellen, Mehrzeller sowie Zellverbände in Frage. Soll die Vorrichtung im Rahmen eines EIA oder ELISA verwendet werden, so werden als Biomoleküle insbesondere spezifische Antikörper verwendet, die in Schritt (f) ortsspezifisch und selektiv durch chemische Bindung über die funktionalen Gruppen des Trockenfotoresistmaterials an mindestens einen Bereich der von dem Trockenfotoresistmaterial gebildeten Innenoberflächen der in Schritt (e) erzeugten Kapillarstruktur gekoppelt bzw. gebunden werden.

In einer bevorzugten Ausführungsform der vorliegenden Erfindung wird in der Kapillarstruktur mindestens eine Kapillare durch Anbinden von Biomolekülen als Affinitätskapillare und mindestens eine Kapillare als Verstärkungskapillare ausgebildet bzw. vorgesehen. Die Funktion der Verstärkungskapillare besteht im wesentlichen darin, das in der Affinitätskapillare bei Durchleiten eines Analyten bzw. einer Probenlösung, wie z.B. einer Serumprobe, erzeugte chemische Signal durch einen Kreisprozess zu verstärken. Vorzugsweise werden die Kanäle der Verstärkungskapillare gleichzeitig mit den Affinitätskapillaren hergestellt, wobei Affinitätskapillare und Verstärkungskapillare direkt miteinander verbunden sind. Pro Affinitätskapillare kann eine Verstärkungskapillare vorgesehen sein. Mehrere Affinitätskapillare können in eine Verstärkungskapillare münden, alternativ kann eine Affinitätskapillare in mehrere Verstärkungskapillaren münden. Ferner können Affinitäts- und Verstärkungskapillare in derselben Ebene angeordnet sein oder separat hergestellt und zur Anwendung vertikal gestapelt werden.

Bei Durchleiten einer zu analysierenden Flüssigkeit (Analyt) wird vorzugsweise das durch Wechselwirkung mit den ortsspezifisch gebundenen Biomolekülen resultierende chemische Signal dann elektrochemisch, vorzugsweise durch amperometrische Messung, oder photo- bzw. fluoreszenzspektroskopisch gemessen.

Wird als chemisches Signal beispielsweise die Freisetzung von Glucose hervorgerufen - und zwar wenn als Biomoleküle, die in Schritt (f) ortsspezifisch und selektiv durch direkte chemische Bindung über die funktionalen Gruppen des Trockenfotoresistmaterials an mindestens einen Bereich der von dem Trockenfotoresistmaterial gebildeten Innenoberflächen der in Schritt (e) erzeugten Kapillarstruktur gekoppelt bzw. gebunden werden, beispielsweise alkalische Phosphatase vorgesehen wird und ein Glukose-6-phoshat enhaltender Analyt durch die Kapillarstruktur geleitet bzw. gepumpt wird - so kann das erzeugte chemische Signal, d.h. beispielsgemäß Glukose, mittels einer oder mehrerer Verstärkungskapillare durch einen Kreisprozeß gemäß nachstehendem Reaktionsschema vielfach umgesetzt und somit in Abhängigkeit der Zyklenanzahl verstärkt werden:

$$\text{Glukose} + O_2 \quad \Longrightarrow (GOD) \Longrightarrow \text{Glukonolacton} + H_2O_2$$

$$\text{Glukonolacton} + NADH \Longrightarrow (GDH) \Longrightarrow \text{Glukose} + NAD^+$$

[GOD = Glukoseoxidase]

[GDH = Glukosedehydrogenase]

Bei jedem Zyklus wird ein Molekül, d.h. beispielsgemäß Glukose, gebildet. Im vorstehenden beispielshaften Reaktionsschema wird $H_2O_2$ gebildet, welches dann an einer Elektrode oxidiert und solcherart quantifiziert wird. Das Ausmaß der durch die Verstärkungskapillare induzierten Signalverstärkung ist dabei im wesentlichen von der Geschwindigkeit der zyklischen Reaktion (ca. das Produkt der jeweiligen Enzymaktivitäten - im obigen Beispiel GOD und GDH), der Zyklenanzahl und der Glukosekonzentration abhängig.

Vorzugsweise wird die Verstärkungskapillare durch die Immobilisierung von mindestens zwei Enzymen, die das chemische Signal der Affinitätskapillare durch einen chemischen Kreisprozess in ein verstärktes chemisches Signal umwandeln, funktionalisiert. Die Immobilisierung der Enzyme an mindestens einen Bereich der von dem Trockenfotoresistmaterial gebildeten Innenoberflächen kann dabei entweder analog zur Beschichtung der Affinitätskapillare durch chemische Bindung über die funktionalen Gruppen des Trockenfotoresistmaterials oder aber durch Einschluss in Membranen bzw. Gele, welche eine oder mehrere der Innenoberflächen der Kapillare bedecken, erfolgen. Diese Membrane bzw. Gele können dabei in besonders vorteilhafter Weise auch photolithografisch erzeugt werden. Die Fixierung der Enzyme mittels Einschluß in ein Gel kann dabei noch höhere Volumenaktivitäten der Enzyme realisieren. Selbstverständlich nimmt dabei ein derartiges Gel nur einen Teil des Kanalquerschnitts ein, um ein Durchpumpen eines entsprechenden Analyten zu gewährleisten. Das Gel ist dabei vorzugsweise an mindestens einen Bereich der von dem Trockenfotoresistmaterial gebildeten Innenoberflächen des Kanalsystems chemisch gebunden. Dies kann durch radikalische Vernetzung des Gels in der Kapillare erreicht werden, wobei noch vorhandene polymerisierbare Gruppen des Trockenfotoresistmaterials an der radikalischen Reaktion teilnehmen.

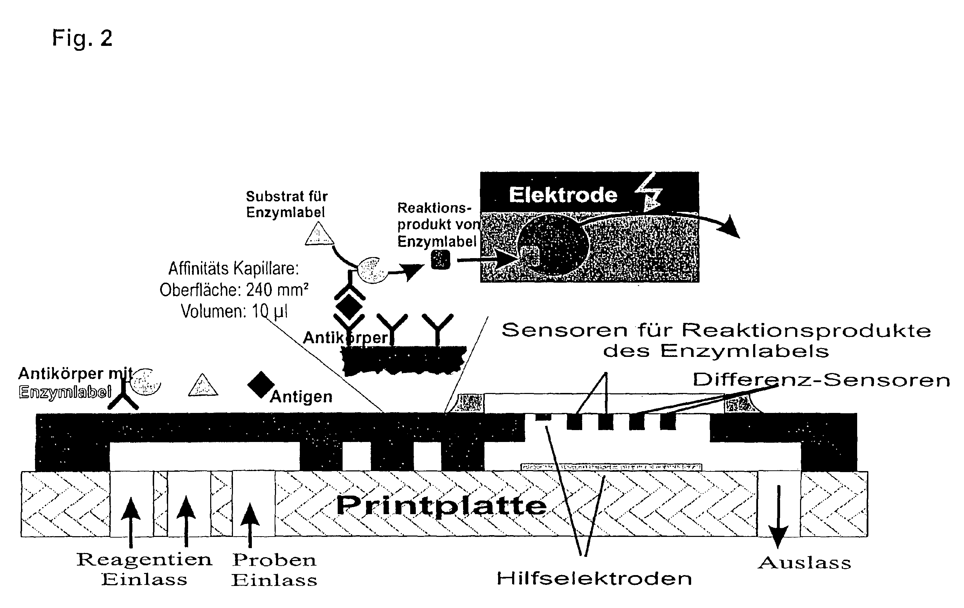

Neben solchen "direkten" Analyseverfahren sind selbstverständlich auch "indirekte" immunologische Bestimmungen biologischer aktiver Substanzen, wie EIA (Enzym-Immunoassay) und ELISA (Enzyme linked immunosorbent assay) im Rahmen der vorliegenden Erfindung durchführbar, wie beispielsweise auch in Fig. 2 gezeigt. Beispielsweise kann die Reaktion zwischen Antigen als die zu messende Substanz einer Probenlösung und spezifischem Antikörper, der in Schritt (f) ortsspezifisch und selektiv durch chemische Bindung über die funktionalen Gruppen des Trockenfotoresistmaterials an mindestens einen Bereich der von dem Trockenfotoresistmaterial gebildeten Innenoberflächen der in Schritt (e) erzeugten Kapillarstruktur gekoppelt bzw. gebunden worden ist, durch nachfolgende Bestimmung eines vorzugsweise an das Antigen gebundenen Enzyms (z.B. Meerrettichperoxidase oder andere pflanzliche, bakterielle oder tierische Enzyme) nachgewiesen werden. Im allgemeinen ist im EIA die Spezifität der Antigen-Antikörper-Reaktion an eine enzymatische Reaktion gekoppelt, indem entweder Antikörper- oder Antigen-Enzym-Konjugate verwendet werden, die nach Zugabe von geeignetem Substrat durch die Messung der Enzym-Aktivität des Konjugats photometrisch, fluorimetrisch oder elektrochemisch bestimmt wird, wobei wiederum Verstärkungskapillare zur Signalverstärkung vorgesehen werden können. Im Rahmen der vorliegenden Erfindung wird beim ELISA der spezifische Antikörper gegen das Antigen als die zu messende Substanz wiederum in Schritt (f) ortsspezifisch und selektiv durch chemische Bindung über die funktionalen Gruppen des Trockenfotoresistmaterials an mindestens einen Bereich der von dem Trockenfotoresistmaterial gebildeten Innenoberflächen der in Schritt (e) erzeugten Kapillarstruktur gekoppelt bzw. gebunden. Daran lagert sich dann das Antigen der Probenlösung, z.B. eine Serumprobe, an und der Probenrest wird abgewaschen. Anschließend wird ein an ein voll wirksames Enzym gekoppelter zweiter Antikörper zugesetzt, der sich an das im ersten Schritt fixierte Antigen bindet. Die Enzym-aktivität des über Antikorper-Antigen-Antikörper mit der Kapillarkanalwand verbundenen Enzyms kann dann nach Zugabe eines entsprechenden Substrats gegebenfalls unter Verwendung einer oder mehrerer Verstärkungskapillare, wie oben ausgeführt, gemessen werden.

Ein weiterer Gegenstand der vorliegenden Erfindung betrifft eine Vorrichtung zur Durchführung von Immunoassays, welche aus mindestens einem Substrat und daran angeordnet mindestens einem Trockenfotoresist-Polymermaterial mit funktionalen chemischen Gruppen aufgebaut ist, wobei Substrat und Trockenfotoresist-Polymermaterial miteinander eine Kapillarstruktur mit mindestens einem Einlass und Auslass bilden, wobei Biomoleküle an die von dem Trockenfotoresistmaterial gebildeten Innenoberflächen der Kapillarstruktur durch chemische Bindung über funktionale Gruppen des Trockenfotoresist-Polymermaterials gebunden sind. Die Trockenfotoresistfilme bzw. -folien, insbesondere solche auf Basis eines negativen Fotoresists, basieren dabei auf den vorgenannten fotostrukturierbaren Polymeren, welche die vorgenannten funktionalen chemischen Gruppen aufweisen. Insbesondere werden Trockenfotoresistfilme auf Basis von Polymermaterialien eingesetzt, die chemische funktionale Gruppen enthalten, ausgewählt aus Carbonsäuren, Carbonsäureanhydriden, Carbonsäurechloriden, Aldehyden, Glyoxalen, N-Hydroxysuccinimidester, Hydraziden, Imidaten, Isothiocyanaten, Isocyanaten, Maleinimiden, Halogenchinonen, Epoxiden, Aziridinen, Acylaziden, Phenolen, Aminogruppen, Thiolgruppen, Hydroxylgruppen, Sulfhydryl-reaktivem Brom und lod sowie Biotingruppen. Vorzugsweise sind die chemischen funktionalen Gruppen Thiolgruppen (-SH) und davon abgeleitete Gruppen wie z.B. Pyridylthiogruppen, Carbonsäuregruppen (-COOH) und davon abgeleitete Gruppen wie beispielsweise Carbonsäureanhydridgruppen wie z.B. Maleinsäureanhydrid oder Bernsteinsäureanhydrid, Imidestergruppen wie z.B. N-Hydroxysuccinimidestergruppen, und Carboxylatgruppen.

In einer bevorzugten Ausführungsform der vorliegenden Erfindung ist der Trokkenfotoresistfilm auf Basis eines Gemisches von Polymeren und gegebenenfalls Oligomeren und/oder Monomeren und weiteren Hilfsstoffen, wie z.B. Füller, Vernetzer, Weichmacher und Photoinitiatoren, wobei mindestens eines der Polymere, Oligomere oder Monomere Thiol-, Carbonsäure-, Anhydrid-, Säureamid- oder Imidestereinheiten, vorzugsweise Acrylsäure-, Methacrylsäure-, Maleinsäureanhydrid-, Maleinsäure-imid- oder N-Hydroxysuccinimidestereinheiten aufweist. Die in einem derartigen Gemisch verwendeten Polymere können Blockpolymere, Copolymere oder Pfropfcopolymere, insbesondere Copolymere, vorzugsweise statistische oder Blockcopolymere sein, die vorzugsweise Carbonsäure-, Anhydrid-, Säureamid- oder Imidestereinheiten enthalten. Im Rahmen der vorliegenden Erfindung ist auch das Vermischen bzw. Compoundieren eines wie vorstehend ausgeführten Trockenfotoresistmaterials mit einem Monomer oder Oligomer eingeschlossen, welches einerseits befähigt ist, in der durch Belichten induzierten Vernetzungsreaktion in das Polymernetzwerk eingebaut zu werden, und andererseits weiter eine der vorstehend angeführten funktionalen chemischen Gruppen aufweist, die befähigt sind, mit entsprechenden Biomolekülen eine chemische Bindung einzugehen. Beispielhaft können hier $\gamma$-Maleimidobutansäure-N-hydroxysuccinimid, $\gamma$-Maleimidocapronsäure-N-hydroxysuccinimid oder N-Acryloxysuccinimid genannt werden.

Als im Rahmen der vorliegenden Erfindung verwendbare Trockenfotoresistfilmmaterialien können hier beispielsweise Vacrel® oder Riston®, beide vertrieben von Dupont, angeführt werden.

Das Substrat bzw. der Träger kann beispielsweise ein Leiter- bzw. Halbleitersubstrat, ein Glassubstrat, ein Kunststoffsubstrat oder ein unstrukturiertes bzw. bereits strukturiertes Fotoresistmaterial sein. Als Biomoleküle können wiederum insbesondere DNA, RNA, PNA, (bei Nukleinsäuren und ihren chemischen Derivaten können z.B. Einzelstränge, Triplex-Strukturen oder Kombinationen hiervon vorliegen), Saccharide, Peptide, Proteine (z.B. Antikörper, Antigene, Rezeptoren), Derivate der kombinatorischen Chemie (z.B. organische Moleküle), Zellbestandteile (z.B. Organellen), Zellen, Mehrzeller sowie Zellverbände angeführt werden. Soll die Vorrichtung im Rahmen eines EIA oder ELISA verwendet werden, so werden als Biomoleküle insbesondere spezifische Antikörper verwendet, die in Schritt (f) ortsspezifisch und selektiv durch chemische Bindung über die funktionalen Gruppen des Trockenfotoresistmaterials an mindestens einen Bereich der von dem Trockenfotoresistmaterial gebildeten Innenoberflächen der in Schritt (e) erzeugten Kapillarstruktur gekoppelt bzw. gebunden werden. In der erfindungsgemäßen Vorrichtung ist innerhalb der Kapillarstruktur vorzugsweise mindestens eine Kapillare durch Anbinden von Biomolekülen als Affinitätskapillare und mindestens eine Kapillare als Verstärkungskapillare ausgebildet. Dabei kann die Verstärkungskapillare beispielsweise durch Immobilisierung von mindestens zwei Enzymen funktionalisiert sein.

In einer bevorzugten Ausführungsform der vorliegenden Erfindung umfasst die Vorrichtung weiter mindestens zwei Elektroden, die pro Kapillare oder für mehrere Kapillare gleichzeitig vorgesehen sein können. Die Elektroden können dabei als Mikroelektroden oder Mikroelektrodenarrays ausgebildet sein. In dieser Ausführungsform wird durch Anordnen der Elektroden eine elektrochemische Detektion zur Quantifizierung des chemischen Signals aus Affinitäts- bzw. Verstärkungskapillare erreicht. Mindestens zwei Elektroden stehen dabei in Kontakt mit der zu analysierenden Flüssigkeit. Die Elektroden, entweder alle oder nur einige, können dabei im Zuge der Herstellung des Kapillarkanalsystems auf dem selben Träger bzw. Substrat hergestellt bzw. angeordnet werden. Alternativ können die Elektroden, entweder alle oder nur einige, getrennt von den Kapillaren hergestellt werden und entweder im Laufe der Herstellung der Vorrichtung oder erst beim Einsatz mit den Kapillaren zusammengeführt werden. Es kann pro Kapillare eine solche Detektionseinheit aus mindestens zwei Elektroden oder alternativ auch eine solche Detektionseinheit für mehrere Kapillaren vorgesehen sein. Vorteilhaft ist inbesondere die amperometrische Messung, bei der das chemische Signal, das beispielsweise in Korrelation zu der Konzentration der gebildeten chemischen Spezies steht, an einer oder mehreren Elektroden, die auf ein konstantes Potential gegen die Messlösung polarisiert sind, elektrochemisch umgesetzt wird, wodurch sich ein messbarer Strom einstellt, der zur Konzentration dieser chemischen Spezies proportional ist. Die Elektroden können dabei vorzugsweise als Mikroelektroden, beispielsweise auch Enzymelektroden, oder Mikroelektrodenarrays ausgebildet sein. Ferner kann das chemische Signal aus der Affinitäts- bzw. Verstärkungskapillare nicht direkt an einer oder mehreren dieser Elektrode umgesetzt werden, sondern mittels über dieser Elektrode in Membranen eingeschlossenen Enzymen noch einer weiteren chemischen Unwandlung und gegebenenfalls noch einer chemischen Verstärkung unterzogen werden. Des weiteren kann die Verwendung einer oder mehrerer zusätzlicher Elektroden, an welcher bzw. welchen die beschriebenen Nachweisprozesse nicht ablaufen und deren Signal zur Differenz-bildung mit dem Signal der anzeigenden Elektrode(n) verwendet wird, wodurch sich das Signal/Rauschverhältnis der Messung steigern lässt, vorgesehen werden.

Alternativ können auch optische, d.h. photometrische Detektionsmethoden vorgesehen sein, insbesonders solche mittels Fluoreszenzmessung. Hierfür wird mindestens ein Teil der Kanalstruktur mit einem optisch transparenten Abschluss versehen.

Zum Betrieb der erfindungsgemäßen Vorrichtung wird zunächst die zu analysierende Flüssigkeit in das Kapillarkanalsystem, d.h. in die mindestens eine Affinitäts-kapillare gebracht. Der Fluß kann entweder für eine gewisse Zeit unterbrochen werden, um den Ablauf der Affinitätsbindungsreaktion, wie z.B. Antikörper-Antigen, zu ermöglichen, oder aber der Fluss wird für eine gewisse Zeit aufrechterhalten, um die Empfindlichkeit durch den Antransport einer größeren Menge an nachzuweisender bzw. zu analysierender Substanz zu steigern. Nach einem Waschschritt wird die Affinitätskapillare mit einer Lösung eines Nachweißbiomoleküls (Protein, Antikörper, DNA, RNA), welches z.B. durch Kopplung mit einem Enzym (z.B. Glukoseoxidase, Meerrettichperoxidase oder andere bakterielle, pflanzliche oder tierische Enzyme) auch katalytische Aktivität besitzt, beschickt. Nach Ablauf der gewünschten Inkubationszeit wird üblicherweise abermals gewaschen und die Affinitätskapillare mit einer Lösung einer Substanz beschickt, welche infolge der katalytischen Aktivität des gebundenen Biomoleküls weiter umgesetzt wird. Die Quantifizierung des Assays kann dann derart erfolgen, dass entweder diese Lösung kontinuierlich durch die Affinitätskapillare sowie gegebenenfalls durch die Verstärkungskapillare und durch die Detektoreinheit gepumpt wird oder alternativ die Lösung für die gewünschte Zeit in der Affinitäts- und/oder der Verstärkungskapillare verbleiben gelassen wird, was zu einer kontinuierlichen Anreicherung der nachzuweisenden Spezies führt, wobei gegebenenfalls das Flüssigkeitsvolumen der Affinitätskapillare in die Verstärkungskapillare transferiert wird, in welcher die Lösung ebenfalls die gewünschte Zeit verbleiben gelassen wird und abschließend dieses Flüssigkeitsvolumen in die Detektionseinheit, welche elektrochemische und/oder photometrische Messeinheiten umfasst, gepumpt wird.

Die Vorrichtung bzw. der Träger für Analytbestimmungsverfahren gemäß der vorliegenden Erfindung eignet sich zur Durchführung von Immunoassays, insbesondere zur immunologischen Bestimmung biologisch aktiver Substanzen in Körperflüssigkeiten. Beispielsweise kann die Reaktion zwischen einem Antikörper, der direkt an die von dem Trockenfotoresistmaterial gebildeten Innenoberflächen der Kapillarstruktur durch chemische Bindung über die funktionale Gruppen des Trockenfotoresist-Polymermaterials gebunden ist, und einem Antigen als die zu messende Substanz nachgewiesen werden, und zwar beispielsweise durch nachfolgende Bestimmung eines an das Antigen oder aber auch den Antikörper gebundenen Enzyms. Derartige Tests eignen sich beispielsweise für Medikamente, Hormone und Proteine.

Ein weiterer Gegenstand der vorliegenden Erfindung betrifft die Verwendung von durch Photolithographie strukturierbaren Trockenfotoresistfilmen auf Basis eines Materials mit funktionalen chemischen Gruppen zur Immobilisierung von Biomolekülen, wobei der Trockenfotoresistfilm insbesondere auf Basis eines negativen Fotoresists ist. Insbesondere werden Trockenfotoresistfilme auf Basis von Polymermaterialien eingesetzt, die chemische funktionale Gruppen enthalten, ausgewählt aus Carbonsäuren, Carbonsäureanhydriden, Carbonsäurechloriden, Aldehyden, Glyoxalen, N-Hydroxysuccinimidester, Hydraziden, Imidaten, Isothiocyanaten, Isocyanaten, Maleinimiden, Halogenchinonen, Epoxiden, Aziridinen, Acylaziden, Phenolen, Aminogruppen, Thiolgruppen, Hydroxylgruppen, Sulfhydryl-reaktivem Brom und Iod sowie Biotingruppen. Vorzugsweise sind die chemischen funktionalen Gruppen Thiolgruppen (-SH) und davon abgeleitete Gruppen wie z.B. Pyridylthiogruppen, Carbonsäuregruppen (-COOH) und davon abgeleitete Gruppen wie beispielsweise Carbonsäureanhydridgruppen wie z.B. Maleinsäureanhydrid oder Bernsteinsäureanhydrid, Imidestergruppen wie z.B. N-Hydroxysuccinimidestergruppen, und Carboxylatgruppen. In einer bevorzugten Ausführungsform der vorliegenden Erfindung ist der Trockenfotoresistfilm auf Basis eines Gemisches von Polymeren und gegebenenfalls Oligomeren und/oder Monomeren und weiteren Hilfsstoffen, wie z.B. Füller, Vernetzer, Weichmacher und Photoinitiatoren, wobei mindestens eines der Polymere, Oligomere oder Monomere Thiol-, Carbonsäure-, Anhydrid-, Säureamid- oder Imidestereinheiten, vorzugsweise Acrylsäure-, Methacrylsäure-, Maleinsäureanhydrid-, Maleinsäureimid- oder N-Hydroxysuccinimidestereinheiten aufweist. Die in einem derartigen Gemisch verwendeten Polymere können Blockpolymere, Copolymere oder Pfropfcopolymere, insbesondere Copolymere, vorzugsweise statistische oder Blockcopolymere sein, die vorzugsweise Carbonsäure-, Anhydrid-, Säureamid- oder Imidestereinheiten enthalten. Im Rahmen der erfindungsgemäßen Verwendung ist auch das Vermischen bzw. Compoundieren eines wie vorstehend ausgeführten Trockenfotoresistmaterials mit einem Monomer oder Oligomer eingeschlossen, welches einerseits befähigt ist, in der durch Belichten induzierten Vernetzungsreaktion in das Polymernetzwerk eingebaut zu werden, und andererseits weiter eine der vorstehend angeführten funktionalen chemischen Gruppen aufweist, die befähigt sind, mit entsprechenden Biomolekülen eine chemische Bindung einzugehen. Beispielhaft können hier γ-Maleimidobutansäure-N-hydroxysuccinimid, γ-Maleimidocapronsäure-N-hydroxysuccinimid oder N-Acryloxysuccinimid genannt werden.

**Patentansprüche**

1. Verfahren zur Herstellung einer Vorrichtung zur Durchführung von Immunoassays, umfassend:

(a) Bereitstellen eines mit vorbestimmten Vertiefungen und/oder Durchgangslöchern mikrostrukturierten Substrats,

(b) Aufbringen von mindestens einem Film eines Trockenfotoresistmaterials mit funktionalen chemischen Gruppen auf das Substrat,

(c) Belichten des Trockenfotoresistfilms unter Verwendung einer Fotoschablone mit einem vorbestimmten Muster,

(d) Entwickeln des Trockenfotoresistfilms,

(e) Wiederholen des Schritts (b), des Schritts (c) unter Verwendung einer Fotoschablone mit einem anderen vorbestimmten Muster und des Schritts (d), so dass Substrat und Trockenfotoresistmaterial miteinander eine Kapillarstruktur mit mindestens einem Einlass und Auslass bilden,

(f) ortsspezifisches Anbinden von Biomolekülen an mindestens einen Bereich der von dem Trockenfotoresistmaterial gebildeten Innenoberflächen der in Schritt (e) erzeugten Kapillarstruktur durch chemische Bindung über die funktionalen Gruppen des Trockenfotoresistmaterials, wodurch eine Vorrichtung zur Durchführung von Immunoassays erhalten wird.

2. Verfahren nach Anspruch 1, wobei der Trockenfotoresistfilm auf Basis eines negativen Fotoresists ist.

3. Verfahren nach Anspruch 1 oder 2, wobei die funktionalen chemischen Gruppen ausgewählt sind aus Carbonsäuren, Carbonsäureanhydriden, Carbonsäurechloriden, Aldehyden, Glyoxalen, N-Hydroxysuccinimidester, Hydraziden, Imidaten, Isothiocyanaten, Isocyanaten, Maleinimiden, Halogenchinonen, Epoxiden, Aziridinen, Acylaziden, Phenolen, Aminogruppen, Thiolgruppen, Hydroxylgruppen, Sulfhydryl-reaktivem Brom und Iod sowie Biotingruppen, vorzugsweise Thiolgruppen (-SH) und davon abgeleiteten Gruppen sowie Carbonsäuregruppen und davon abgeleiteten Gruppen.

4. Verfahren nach einem der Ansprüche 1 bis 3, wobei der Trockenfotoresistfilm auf Basis eines Gemisches von Polymeren und gegebenenfalls Oligomeren und/oder Monomeren ist, wobei mindestens eines der Polymere, Oligomere oder Monomere Thiol-, Carbonsäure-, Anhydrid-, Säureamid- oder Imidestereinheiten, vorzugsweise Acrylsäure-, Methacrylsäure-, Maleinsäureanhydrid-, Maleinsäureimid- oder N-Hydroxysuccinimidestereinheiten, aufweist.

5. Verfahren nach einem der Ansprüche 1 bis 4, worin vor dem Schritt (f) die funktionalen chemischen Gruppen aktiviert werden.

6. Verfahren nach einem der Ansprüche 3 bis 5, worin die Aktivierung, wenn die funktionalen Gruppen Carbonsäuregruppen sind, durch Umsetzung mit einer Carbodiimid-Verbindung durchgeführt wird.

7. Verfahren nach einem der Ansprüche 1 bis 6, worin der Schritt (f) mittels Tauch- oder Gießverfahren durchgeführt wird und anschließend die Kanäle der Kapillarstruktur mit einer oberen Lage von mindestens einem Trockenfotoresistfilm oder einer selbstklebenden Folie verschlossen werden.

8. Verfahren nach einem der Ansprüche 1 bis 6, worin im Zuge des Schritts (e) mit Ausnahme der als Einlässe und Auslässe vorgesehenen Kanäle die Kapillarstruktur verschlossen wird und anschließend der Schritt (f) mittels Pumptechniken durchgeführt wird.

9. Verfahren nach einem der vorhergehenden Ansprüche, worin in Schritt (f) eine Lösung von Biomolekülen unter Ausnutzen der Kapillarkraft mittels mindestens einer Nadel oder Nadelanordnung in eine oder mehrere Kapillare der in Schritt (e) erhaltenen Kapillarstruktur beschickt wird.

10. Verfahren nach einem der vorhergehenden Ansprüche, worin in Schritt (e) als Biomoleküle DNA, RNA, PNA, Saccharide, Peptide, Proteine, Zellbestandteile, Zellen, Mehrzeller sowie Zellverbände eingesetzt werden.

11. Verfahren nach einem der vorhergehenden Ansprüche, worin in der Kapillarstruktur mindestens eine Kapillare durch Anbinden von Biomolekülen als Affinitätskapillare und mindestens eine Kapillare als Verstärkungskapillare ausgebildet wird.

12. Verfahren nach Anspruch 11, worin die Verstärkungskapillare durch Immobilisierung von mindestens zwei Enzymen funktionalisiert wird.

13. Verfahren nach Anspruch 12, worin die Immobilisierung der Enzyme an mindestens einen Bereich der von dem Trockenfotoresistmaterial gebildeten Innenoberflächen durch chemische Bindung über die funktionalen Gruppen des Trockenfotoresistmaterials oder durch Einschluss in Membranen, welche eine oder mehrere der Innenoberflächen der Kapillarstruktur bedecken, durchgeführt wird.

14. Verfahren nach Anspruch 13, worin die Membran photolithographisch hergestellt wird.

15. Verfahren nach einem der vorhergehenden Ansprüche, worin bei Durchleiten einer zu analysierenden Flüssigkeit das durch Wechselwirkung mit den ortsspezifisch gebundenen Biomolekülen resultierende chemische Signal elektrochemisch, vorzugsweise durch amperometrische Messung, oder fluoreszenzspektroskopisch gemessen wird.

16. Vorrichtung zur Durchführung von Immunoassays, welche aus mindestens einem Substrat und daran angeordnet mindestens einem Trockenfotoresist-Polymermaterial mit funktionalen chemischen Gruppen aufgebaut ist, wobei Substrat und Trockenfotoresist-Polymermaterial miteinander eine Kapillarstruktur mit mindestens einem Einlass und Auslass bilden, wobei Biomoleküle an die von dem Trockenfotoresistmaterial gebildeten Innenoberflächen der Kapillarstruktur durch chemische Bindung über funktionale Gruppen des Trockenfotoresist-Polymermaterials gebunden sind.

17. Vorrichtung nach Anspruch 16, wobei der Trockenfotoresistfilm auf Basis eines negativen Fotoresists ist.

18. Vorrichtung nach Anspruch 16 oder 17, wobei die funktionalen chemischen Gruppen ausgewählt sind aus Carbonsäuren, Carbonsäureanhydriden, Carbonsäurechloriden, Aldehyden, Glyoxalen, N-Hydroxysuccinimidester, Hydraziden, Imidaten, Isothiocyanaten, Isocyanaten, Maleinimiden, Halogenchinonen, Epoxiden, Aziridinen, Acylaziden, Phenolen, Aminogruppen, Thiolgruppen, Hydroxylgruppen, Sulfhydryl-reaktivem Brom und Iod sowie Biotingruppen, vorzugsweise Thiolgruppen (-SH) und davon abgeleiteten Gruppen sowie Carbonsäuregruppen und davon abgeleiteten Gruppen.

19. Vorrichtung nach einem der Ansprüche 16 bis 18, wobei der Trockenfotoresistfilm auf Basis eines Gemisches von Polymeren und gegebenenfalls Oligomeren und/oder Monomeren ist, wobei mindestens eines der Polymere, Oligomere oder Monomere Thiol-, Carbonsäure-, Anhydrid-, Säureamid- oder Imidestereinheiten, vorzugsweise Acrylsäure-, Methacrylsäure-, Maleinsäureanhydrid-, Maleinsäureimid- oder N-Hydroxysuccinimidestereinheiten, aufweist.

20. Vorrichtung nach einem der Ansprüche 16 bis 19, worin die Biomoleküle ausgewählt sind aus DNA, RNA, PNA, Sacchariden, Peptiden, Proteinen, Zellbestandteilen, Zellen, Mehrzeller sowie Zellverbänden.

21. Vorrichtung nach einem der Ansprüche 16 bis 20, wobei in der Kapillarstruktur mindestens eine Kapillare durch Anbinden von Biomolekülen als Affinitätskapillare und mindestens eine Kapillare als Verstärkungskapillare ausgebildet ist.

22. Vorrichtung nach Anspruch 21, wobei die Verstärkungskapillare durch Immobilisierung von mindestens zwei Enzymen funktionalisiert ist.

23. Vorrichtung nach einem der Ansprüche 16 bis 22, weiter umfassend mindestens zwei Elektroden, die pro Kapillare oder für mehrere Kapillare vorgesehen sein können.

24. Vorrichtung nach Anspruch 23, wobei die Elektroden als Mikroelektroden oder Mikroelektrodenarrays ausgebildet sind.

25. Verwendung der Vorrichtung nach einem der Ansprüche 16 bis 24 zur Durchführung von Immunoassays.

26. Verwendung von durch Photolithographie strukturierbaren Trockenfotoresistfilmen auf Basis eines Materials mit funktionalen chemischen Gruppen zur Immobilisierung von Biomolekülen.

27. Verwendung nach Anspruch 26, wobei der Trockenfotoresistfilm auf Basis eines negativen Fotoresists ist.

28. Verwendung nach Anspruch 26 oder 27, wobei die funktionalen chemischen Gruppen ausgewählt sind aus Carbonsäuren, Carbonsäureanhydriden, Carbonsäurechloriden, Aldehyden, Glyoxalen, N-Hydroxysuccinimidester, Hydraziden, Imidaten, Isothiocyanaten, Isocyanaten, Maleinimiden, Halogenchinonen, Epoxiden, Aziridinen, Acylaziden, Phenolen, Aminogruppen, Thiolgruppen, Hydroxylgruppen, Sulfhydryl-reaktivem Brom und Iod sowie Biotingruppen, vorzugsweise Thiolgruppen (-SH) und davon abgeleiteten Gruppen sowie Carbonsäuregruppen und davon abgeleiteten Gruppen.

29. Verwendung nach einem der Ansprüche 26 bis 28, wobei der Trockenfotoresistfilm auf Basis eines Gemisches von Polymeren und gegebenenfalls Oligomeren und/oder Monomeren ist, wobei mindestens eines der Polymere, Oligomere oder Monomere Thiol-, Carbonsäure-, Anhydrid-, Säureamid- oder Imidestereinheiten, vorzugsweise Acrylsäure-, Methacrylsäure-, Maleinsäureanhydrid-, Maleinsäureimid- oder N-Hydroxysuccinimidestereinheiten, aufweist.

**Claims**

1. A method for the fabrication of a device for performing immuno assays comprising:

   (a) providing a micro structured substrate having predefined indentations and/or through holes,
   (b) applying at least one layer of a dry film photo resist material with functional chemical groups onto said substrate,
   (c) light exposing the dry film photo resist using a photo mask with a different predefined pattern,
   (d) developing the dry film photo resist layer,
   (e) repeating step (b) and step (c) using a photo mask with a different predefined pattern and step (d), so that substrate and dry film photo resist material form a capillary structure or capillary channel structure, respectively, with at least one inlet and outlet,
   (f) localized immobilizing biomolecules to at least one region of the inner surface of the dry film photo resist material channel structure generated in step (e) by chemical coupling via the functional groups of the dry film photo resist material, wherein a device for performing immuno assays is obtained.

2. The method according to claim 1, wherein the dry film photo resist is based on a negative photo resist.

3. The method according to claim 1 or 2, wherein the functional chemical groups are selected from carboxylic acids, carboxylic acids anhydrids, carboxylic acid chlorides, aldehydes, glyoxals, N-hydroxy succinimide esters, hydrazides, imidates, isothiocyanates, isocyanates, maleinimides, halogenquinones, epoxides, aziridines, acylazides, phenoles, amino groups, thiol groups, hydroxyl groups, sulfhydryl-reactive bromium and iodine and biotin groups, preferably thiol groups (-SH) and groups derived therefrom and carboxylic acid groups (-COOH) and groups derived therefrom.

4. The method according to any one of claims 1 to 3, wherein the dry film photo resist is based on a mixture of polymers and optionally oligomers and/or monomers, wherein at least one of the polymers, oligomers or monomers comprises thiol-, carboxylic acid-, anhydride-, acid-amide- or imide ester groups, preferably acrylic acid-, methacrylic acid-, maleic acid anhydride-, maleic acid imide- or N-hydroxy succinimide ester groups.

5. The method according to any one of claims 1 to 4, wherein the functional chemical groups are activated before step (f).

6. The method according to any one of claims 3 to 5, wherein the activation, if the functional groups are carboxylic acid groups, is carried out by reaction with a carbodiimide compound.

7. The method according to any one of claims 1 to 6, wherein step (f) is performed by using an immersion or pouring process and the channels of the capillary structure are subsequently closed with either a top layer of at least one dry film photo resist layer or with a self sealing film.

8. The method according to any one of claims 1 to 6, wherein in the course of step (e) the predefined channels of the capillary structure are closed, with the exception of the channels provided as inlets and outlets, and subsequently step (f) is performed by using pumping techniques.

9. The method according to any one of the preceding claims, wherein in step (f) a solution of biomolecules is delivered to one or more capillaries of the capillary structure obtained in step (e) by means of at least one needle or an arrangement of needles utilizing capillary forces.

10. The method according to any one of the preceding claims, wherein the biomolecules used in step (e) are DNA, RNA, PNA, saccharides, peptides, proteines, cellular components, cells, multicellular organisms and cell assemblies.

11. The method according to any one of the preceding claims, wherein by binding of biomolecules, at least one capillary of the capillary structure is made as affinity capillary and at least one capillary is made as amplification capillary.

12. The method according to claim 11, wherein the amplification capillary is functionalized by immobilization of at least two enzymes.

13. The method according to claim 12, wherein the immobilization of the enzymes to at least one region of the inner surface of the capillary structure formed by the dry film photo resist material, is carried out by chemical linking via the functional groups of the dry film photo resist material or by entrapment into a membrane that covers one or more of the inner surfaces of the capillary structure.

14. The method according to claim 13, wherein the membrane is fabricated photo lithographically.

15. The method according to any one of the preceding claims, wherein the chemical signal, generated due to the interaction of the bound biomolecules with a passing fluid to be analyzed, is measured electrochemically, preferably amperometrically, or fluorescence spectrophotometrically.

16. A device for performing immuno assays, comprising at least one substrate and provided thereon at least one dry film photo resist polymer material layer comprising functional chemical groups, wherein substrate and dry film photo resist polymer material together form a capillary structure with at least one inlet and one outlet, wherein biomolecules are bound to at least one region of the inner surface of the capillary structure, formed by the dry film photo resist material, by a chemical link via the functional groups of the dry film photo resist material.

17. The device according to claim 16, wherein the dry film photo resist is based on a negative photo resist.

18. The device according to claim 16 or 17, wherein the functional chemical groups are selected from carboxylic acids, carboxylic acids anhydrids, carboxylic acid chlorides, aldehydes, glyoxals, N-hydroxy succinimide esters, hydrazides, imidates, isothiocyanates, isocyanates, maleinimides, halogenquinones, epoxides, aziridines, acylazides, phenoles, amino groups, thiol groups, hydroxyl groups, sulfhydryl-reactive bromium and iodine and biotin groups, preferably thiol groups (-SH) and groups derived therefrom and carboxylic acid groups (-COOH) and groups derived therefrom.

19. The device according to any one of claims 16 to 18, wherein the dry film photo resist is based on a mixture of polymers and optionally oligomers and/or monomers, wherein at least one of the polymers, oligomers or monomers comprises thiol-, carboxylic acid-, anhydride-, acid-amide- or imide ester groups, preferably acrylic acid-, methacrylic acid-, maleic acid anhydride-, maleic acid imide- or N-hydroxy succinimide ester groups.

20. The device according to any one of claims 16 to 19, wherein the biomolecules are selected from DNA, RNA, PNA, saccharides, peptides, proteines, cellular components, cells, multicellular organisms and cell assemblies.

21. The device according to any one of claims 16 to 20, wherein, by binding of biomolecules, at least one capillary of the capillary structure is made as affinity capillary and at least one capillary as made as amplification capillary.

22. The device according to claim 21, wherein the amplification capillary is functionalized by immobilization of at least two enzymes.

23. The device according to any one of claims 16 to 22, further comprising at least two electrodes that can be adopted for one or for more capillaries.

24. The device according to claim 23, wherein the electrodes are designed as micro electrodes or micro electrode arrays.

25. Use of the device according to any one of claims 16 to 24 for performing immuno assays.

26. Use of a photo lithographically patternable dry film photo resist based on a material with functional chemical groups for the immobilization of biomolecules.

27. The use according to claim 26, wherein the dry film photo resist is based on a negative photo resist.

28. The use according to claim 26 or 27, wherein the functional chemical groups are selected from carboxylic acids, carboxylic acids anhydrids, carboxylic acid chlorides, aldehydes, glyoxals, N-hydroxy succinimide esters, hydrazides, imidates, isothiocyanates, isocyanates, maleinimides, halogenquinones, epoxides, aziridines, acylazides, phenoles, amino groups, thiol groups, hydroxyl groups, sulfhydryl-reactive bromium and iodine and biotin groups, preferably thiol groups (-SH) and groups derived therefrom and carboxylic acid groups (-COOH) and groups derived therefrom.

29. The use according to any one of claims 26 to 28, wherein the dry film photo resist is based on a mixture of polymers and optionally oligomers and/or monomers, wherein at least one of the polymers, oligomers or monomers comprises thiol-, carboxylic acid-, anhydride-, acid-amide- or imide ester groups, preferably acrylic acid-, methacrylic acid-, maleic acid anhydride-, maleic acid imide- or N-hydroxy succinimide ester groups.

**Revendications**

1. Procédé de fabrication d'un dispositif de réalisation de dosages immunologiques, comprenant :

   (a) la préparation d'un substrat microstructuré avec des renfoncements prédéterminés et/ou des orifices de passage,
   (b) l'application d'au moins un film d'une laque photosensible sèche avec des groupements chimiques fonctionnels sur le substrat,
   (c) l'exposition à la lumière du film de laque photosensible sèche en utilisant un gabarit d'éclairage avec un échantillon prédéterminé,
   (d) le développement du film de laque photosensible sèche,
   (e) la répétition de l'étape (b), de l'étape (c) en utilisant un gabarit d'éclairage avec un autre échantillon prédéterminé, et de l'étape (d), de sorte que le substrat et le film de laque photosensible sèche forment l'un avec l'autre une structure capillaire avec au moins un orifice d'entrée et un orifice de sortie,
   (f) l'attachement topologiquement spécifique de biomolécules à au moins un domaine des surfaces internes formées par la laque photosensible sèche de la structure capillaire produite à l'étape (e) par liaison chimique sur les groupements fonctionnels de la laque photosensible sèche, de sorte que l'on obtient un dispositif de réalisation de dosages immunologiques.

2. Procédé selon la revendication 1, dans lequel le film de laque photosensible sèche est à base d'une laque photosensible négative.

3. Procédé selon la revendication 1 ou 2, dans lequel les groupements chimiques fonctionnels sont choisis parmi des acides carboxyliques, des anhydrides carboxyliques, des chlorures d'acyles, des aldéhydes, des glyoxals, des esters de N-hydroxysuccinimides, des hydrazines, des imidates, des isothiocyanates, des isocyanates, des imides maléiques, des halogénochinones, des époxydes, des aziridines, des acylazides, des phénols, des groupements amines, des groupements thiols, des groupements hydroxyles, des bromes et des iodes réactifs aux sulfhydryles, ainsi que des groupements biotines, de préférence des groupements thiols (-SH) et des groupements dérivés de ces derniers ainsi que des groupements acides carboxyliques et des groupements dérivés de ces derniers.

4. Procédé selon l'une des revendications 1 à 3, dans lequel le film de laque photosensible sèche est à base d'un mélange de polymères et le cas échéant d'oligomères et/ou de monomères, dans lequel au moins un des polymères, oligomères ou monomères présente des unités thiols, acides carboxyliques, anhydrides, amides ou imidesters, de préférence des unités acides acryliques, acides méthacryliques, anhydrides maléiques, imides maléiques ou esters de N-hydroxysuccinimides.

5. Procédé selon l'une des revendications 1 à 4, dans lequel avant l'étape (f), les groupements chimiques fonctionnels sont activés.

6. Procédé selon l'une des revendications 3 à 5, dans lequel l'activation, quand les groupements fonctionnels sont des acides carboxyliques, est effectuée par réaction avec un composé carbodiimide.

7. Procédé selon l'une des revendications 1 à 6, dans lequel l'étape (f) est effectuée au moyen d'un procédé par trempage ou par coulée, à la suite de quoi les canaux de la structure capillaire sont fermés avec une couche supérieure d'au moins un film de laque photosensible sèche ou une feuille auto-adhésive.

8. Procédé selon l'une des revendications 1 à 6, dans lequel au cours de l'étape (e), à l'exception des canaux prévus comme entrées et sorties, la structure capillaire est fermée, à la suite de quoi l'étape (f) est effectuée au moyen de techniques de pompage.

9. Procédé selon l'une des revendications précédentes, dans lequel, au cours de l'étape (f), une solution de biomolécules est envoyée dans un ou plusieurs capillaires de la structure capillaire obtenue à l'étape (e) par utilisation de la force de capillarité au moyen d'au moins une aiguille ou d'un ensemble d'aiguilles.

10. Procédé selon l'une des revendications précédentes, dans lequel, au cours de l'étape (e), on utilise comme biomolécules de l'ADN, de l'ARN, des PNA, des saccharides, des peptides, des protéines, des composants cellulaires, des cellules, des organismes pluricellulaires, ainsi que des assemblages cellulaires.

11. Procédé selon l'une des revendications précédentes, dans lequel, dans la structure capillaire, au moins un capillaire est formé par attachement de biomolécules comme capillaire d'affinité et au moins un capillaire est formé comme capillaire de renforcement.

12. Procédé selon la revendication 11, dans lequel le capillaire de renforcement est fonctionnalisé par immobilisation d'au moins deux enzymes.

13. Procédé selon la revendication 12, dans lequel l'immobilisation des enzymes à au moins un domaine des surfaces internes formées par la laque photosensible sèche est effectuée par liaison chimique sur les groupements fonctionnels de la laque photosensible sèche ou par inclusion dans des membranes qui recouvrent une ou plusieurs des surfaces internes de la structure capillaire.

14. Procédé selon la revendication 13, dans lequel la membrane est fabriquée par photolithographie.

15. Procédé selon l'une des revendications précédentes, dans lequel, pendant le passage du liquide à analyser, le signal chimique qui résulte de l'interaction avec les biomolécules liées de façon topologiquement spécifique est mesuré électrochimiquement, de préférence par mesure ampérométrique, ou par spectroscopie de fluorescence.

16. Dispositif de réalisation de dosages immunologiques, lequel est conçu à partir d'au moins un substrat et d'au moins une laque photosensible sèche disposée dessus avec des groupements chimiques fonctionnels, dans lequel le substrat et la laque photosensible sèche forment l'un avec l'autre une structure capillaire avec au moins un orifice d'entrée et un orifice de sortie, dans lequel des biomolécules sont liées aux surfaces internes de la structure capillaire formées par la laque photosensible sèche par liaison chimique sur les groupements fonctionnels de la laque photosensible sèche.

17. Dispositif selon la revendication 16, dans lequel le film de laque photosensible sèche est à base d'une laque photosensible négative.

18. Dispositif selon la revendication 16 ou 17, dans lequel les groupements chimiques fonctionnels sont choisis parmi des acides carboxyliques, des anhydrides carboxyliques, des chlorures d'acyles, des aldéhydes, des glyoxals, des esters de N-hydroxysuccinimides, des hydrazines, des imidates, des isothiocyanates, des isocyanates, des imides maléiques, des halogénochinones, des époxydes, des aziridines, des acylazides, des phénols, des groupements amines, des groupements thiols, des groupements hydroxyles, des bromes et des iodes réactifs aux sulfhydryles, ainsi que des groupements biotines, de préférence des groupements thiols (-SH) et des groupements dérivés de ces derniers, ainsi que des groupements acides carboxyliques et des groupements dérivés de ces derniers.

19. Dispositif selon l'une des revendications 16 à 18, dans lequel le film de laque photosensible sèche est à base d'un mélange de polymères et le cas échéant d'oligomères et/ou de monomères, dans lequel au moins un des polymères, oligomères ou monomères présente des unités thiols, acides carboxyliques, anhydrides, amides ou imidesters, de préférence des unités acides acryliques, acides méthacryliques, anhydrides maléiques, imides maléiques ou esters de N-hydroxysuccinimides.

20. Dispositif selon l'une des revendications 16 à 19, dans lequel les biomolécules sont choisies parmi des ADN, des ARN, des PNA, des saccharides, des peptides, des protéines, des composants cellulaires, des cellules, des organismes pluricellulaires, ainsi que des assemblages cellulaires.

21. Dispositif selon l'une des revendications 16 à 20, dans lequel, dans la structure capillaire, au moins un capillaire est formé par attachement de biomolécules comme capillaire d'affinité et au moins un capillaire est formé comme capillaire de renforcement.

22. Dispositif selon la revendication 21, dans lequel le capillaire de renforcement est fonctionnalisé par immobilisation d'au moins deux enzymes.

23. Dispositif selon l'une des revendications 16 à 22, comprenant en outre au moins deux électrodes qui peuvent être prévues par capillaire ou pour plusieurs capillaires.

24. Dispositif selon la revendication 23, dans lequel les électrodes sont constituées comme des microélectrodes ou des ensembles de microélectrodes.

25. Utilisation du dispositif selon l'une des revendications 16 à 24 pour la réalisation de dosages immunologiques.

26. Utilisation de films de laque photosensible sèche structurables par photolithographie à base d'un matériau avec des groupements chimiques fonctionnels pour l'immobilisation de biomolécules.

27. Utilisation selon la revendication 26, dans laquelle le film de laque photosensible sèche est à base d'une laque photosensible négative.

28. Utilisation selon la revendication 26 ou 27, dans laquelle les groupements chimiques fonctionnels sont choisis parmi des acides carboxyliques, des anhydrides carboxyliques, des chlorures d'acyles, des aldéhydes, des glyoxals, des esters de N-hydroxysuccinimides, des hydrazines, des imidates, des isothiocyanates, des isocyanates, des imides maléiques, des halogénochinones, des époxydes, des aziridines, des acylazides, des phénols, des groupements amines, des groupements thiols, des groupements hydroxyles, des bromes et des iodes réactifs aux sulfhydryles, ainsi que des groupements biotines, de préférence des groupements thiols (-SH) et des groupements dérivés de ces derniers, ainsi que des groupements acides carboxyliques et des groupements dérivés de ces derniers.

29. Utilisation selon l'une des revendications 26 à 28, dans laquelle le film de laque photosensible sèche est à base d'un mélange de polymères et le cas échéant d'oligomères et/ou de monomères, dans laquelle au moins un des polymères, oligomères ou monomères présente des unités thiols, acides carboxyliques, anhydrides, amides ou imidesters, de préférence des unités acides acryliques, acides méthacryliques, anhydrides maléiques, imides maléiques ou esters de N-hydroxysuccinimides.

# Fig. 1

**Verarbeiten eines Films auf Basis eines negativen Trockenfotoresists**

Laminieren | UV-Bestrahlung | Entwicklung

Fig. 2

Substrat für
Enzymlabel

Reaktionsprodukt von
Enzymlabel

**Elektrode**

Affinitäts Kapillare:
Oberfläche: 240 mm²
Volumen: 10 µl

Antikörper

Sensoren für Reaktionsprodukte
des Enzymlabels

Antikörper mit
Enzymlabel

Antigen

Differenz-Sensoren

**Printplatte**

Reagentien
Einlass

Proben
Einlass

Hilfselektroden

Auslass

EP 1 417 492 B1